(19) **Europäisches Patentamt**

**European Patent Office**

**Office européen des brevets**

(11) Publication number: **0 346 500 B1**

(12) **EUROPEAN PATENT SPECIFICATION**

(45) Date of publication of patent specification: **27.07.94**

(21) Application number: **89900924.5**

(22) Date of filing: **28.12.88**

(86) International application number:
**PCT/JP88/01342**

(87) International publication number:
**WO 89/06239 (13.07.89 89/15)**

The file contains technical information submitted after the application was filed and not included in this specification

(51) Int. Cl.⁵: **C07K 3/08**, C07K 13/00, C07K 15/12, C12P 21/02, C12N 15/00, C12N 9/99, A61K 37/02, //(C12P21/02, C12R1:19)

(54) **ELASTASE INHIBITING POLYPEPTIDES AND PROCESS FOR THEIR PREPARATION BY GENETIC RECOMBINATION.**

(30) Priority: **28.12.87 JP 330219/87**

(43) Date of publication of application:
**20.12.89 Bulletin 89/51**

(45) Publication of the grant of the patent:
**27.07.94 Bulletin 94/30**

(84) Designated Contracting States:
**AT BE CH DE FR GB IT LI NL SE**

(56) References cited:
**JP-A-62 501 262**
**JP-A-62 529 591**

(73) Proprietor: **TEIJIN LIMITED**
**11 Minamihonmachi 1-chome**
**Higashi-ku**
**Osaka-shi Osaka 541(JP)**

(72) Inventor: **SUGIYAMA, Takashi**
**3-18-4, Tamadaira**
**Hino-shi Tokyo 191(JP)**
Inventor: **KAMIMURA, Takashi**
**2-7-12, Shinmei**
**Hino-shi Tokyo 191(JP)**
Inventor: **MASUDA, Kenichi**
**2-23-8, Myojin-cho**
**Hachioji-shi Tokyo 192(JP)**
Inventor: **OKADA, Masahiro**
**Teijin Musashino Ryo**
**3-5-18, Tamadaira**
**Hino-shi Tokyo 191(JP)**

EP 0 346 500 B1

Ohlsson et al.: Structure, Genomic Organization, and Tissue Distribution of Human Secretory Leukocyte- Protease Inhibitor (SLPI): A Potent Inhibitor of Neutrophil Elastase; Pulm. Emphysema Proteolysis (Conf.), Meeting date 1986, Edited by: Taylor J,C. et al., Academic: Orlando, FLA, pp.: 307-322 (1987)

Stetler et al.: Isolation and Sequence of a Human Gene Encoding a Potent Inhibitor of Leukozyte Proteases, Nucleic Acids Res., Vol: 83(18), pp.: 6692-6696 (1986)

Thompson et al.: Isolation, Properties and Complete Amino Acid Sequence of Human Secretory Leukocyte Protease Inhibitor, Proc.Natl.Acad.Sci., Vol: 83(18), pp.: 6692-6696 (1986)

Inventor: OHTSUKA, Eiko
18-1-3-614, Nishi
Minamijyujyo, Chuo-ku
Sapporo-shi Hokkaido 064(JP)

(74) Representative: Dean, John Paul et al
Withers & Rogers
4 Dyer's Buildings
Holborn
London EC1N 2JT (GB)

**Description**

FIELD OF INVENTION

The present invention relates to an elastase inhibitory polypeptide and a fused protein suitable for the production thereof, as well as a process for the production of the elastase inhibitory polypeptide from this fused protein. Moreover, the present invention relates to a fused protein gene used for the production of said fused protein, a expression plasmid, and a microorganism carrying the same.

BACKGROUND ART

Advances in recombinant DNA techniques have made it possible to produce clinically or economically valuable proteins (hereinafter designated as desired protein) in microbial cells. When such proteins are produced by using microbial cells as host cells, first a gene coding for a desired protein is isolated from various cells or chemically synthesized, and then the gene is expressed in host cells, resulting in the production of the desired protein. Nevertheless, obstacles to or problems in the expression of the gene and production of the desired protein exist, and therefore, to produce the desired protein or a portion thereof in microbial cells efficiently and in a commercially liable scale, first the above-mentioned problems must be solved.

For example, where it is intended to produce a protein having a relatively low molecular weight, such as a peptide or protein having not more than 100 amino acid residues, the produced peptide or protein is recognized as a foreign substance in microbial cells, and tends to be hydrolized by various kinds of proteolytic enzymes, and this often makes it impossible to efficiently produce the desired protein. Therefore, to solve such problems, various approaches have been attempted.

As one of these approaches, it is known that a fused protein gene is constituted by linking a gene for a desired protein and a gene for a protein other than the desired protein (designated as carrier protein) followed by an expression of the fused protein gene in microbial cells to produce the desired protein.

As an embodiment thereof, a process is known wherein a gene for coding a desired protein is joined with an endogeneous gene coding for a microbial cellular protein or a portion thereof, and the constructed gene is expressed in microbial cells. In this process, a fused protein comprising a desired protein fused with the entire microbial cellular protein or a portion thereof, which is not intracellularly hydrolyzed, is able to be obtained. Such microbial protein used as carrier proteins for the above-mentioned purpose include $\beta$-galactosidase (S. Tanaka et al., Nucl. Acids. Res. $\underline{10}$, 1741 - 1754, 1982), $\beta$-lactamase (P. Cornelis et al., Mol. Gen. Genet. $\underline{186}$, 507-511, 1982), chloramphenical acethyl transferase (A. Hobden et al., WPI 87-88509/13), alkaline phosphatase (Japanese Unexamined Patent Publication No. 58-225098). Moreover, as an example of a carrier protein which not only protects a desired protein from a proteolytic degradation but also makes a purification of the desired protein easier, Staphylococcus protein A is known (Japanese Unexamined Patent Publication No. 62-190087; T. Moks et al., Biochemistry $\underline{26}$, 5239 - 5244, 1987).

As a second embodiment thereof, a process is known wherein a gene encoding a desired protein is linked with a gene of an exogeneous protein or a protein thereof, which is a foreign protein itself but is not recognized a stranger in the cells, is expressed. In this process, an exogeneous protein or a portion thereof fused to the desired protein is obtained stable at high levels. Examples of such exogeneous proteins are $\beta$-interferon (I. Ivanov et al., FEBS Lett. $\underline{210}$. 56-60, 1987), $\alpha_1$-antitrypsin (Van der Straten A et al Bioscience Reports $\underline{6}$ 363-373, 1986) and so on.

Fused proteins thus obtained comprising a desired protein and carrier protein sometimes completely lose the biological activity of the desired protein or the biological activity of the desired protein is reduced. Alternatively, even if fused proteins have a biological activity of the desired protein they may exhibit biological activities of the carrier protein such as antigenity, and therefore, the fused proteins as such cannot be clinically used. Accordingly, the desired protein must be cleaved from the fused protein. To this end, an amino acid sequence of a junction (hereinafter designated as a linking peptide) between a carrier protein and a desired protein is successfully designed so that the junction is site-specifically cleaved, and after the production of the fused protein in cells, the desired protein is cleaved from the fused protein.

Generally, a fused protein produced in microbial cells is designed so that a carrier protein constitutes an N-terminal portion of a fused protein. Such a structure can provide a desired protein free from both the carrier protein and N-terminal amino acid residue, methionine, by cleavage at a linking peptide, and therefore, is clinically advantageous.

Such site-specific cleavage methods include chemical cleavage methods using chemical reagents and biological cleavage methods using enzymes. The chemical cleavage methods include the cyanogen

3

bromide method, hydroxylamine method, formic acid method, NBS (N-bromosuccinimide method), lithium/methylamine/NBS method, bromine/hydrochloric acid method, and the like.

The cyanogen bromide method is most often used, and Itakura et al. is succeeded in generating a desired protein, somatostatin, from Escherichia coli β-galactosidase fused somatostatin using cyanogen bromide (K. Itakura et al., Science 198, 1056, 1977; Japanese Unexamined Patent Publication No. 54-163600). Cyanogen bromide hydrolyzes a peptide bond at methionine residue under an article condition. Therefore, to site-specifically cleave a fused protein, the desired protein must have a methionine residue at its N-terminus and contain no methionine residue in an amino acid sequence of the desired protein, and therefore, the application of the cyanogen bromide is necessarily limited.

Enzymatic methods include the endopeptidase method. Endopeptidase recognizes one or more than one specific amino acid in an internal amino acid sequence, and preferentially cleaves a peptide bond at a carboxyl site of a specific amino acid. Herein an amino acid or amino acid sequence which is specifically recognised by endopeptidase is designated as a "recognised amino acid" or "recognised amino acid sequence". The various endopeptidases used to cleave a fused protein produced by microbial cells to generate a desired protein include the following: trypsin used to generate human calcitonin from tryptophan synthetase (WO 84/00380); trypsin used to generate β-endorphin from a β-galactosidase fused 48,3 -endorphin; bovine enteropeptidase used to generate enkephalin from β-galactosidase fused enkephalin (V. N. Dobrynin et al., Bioory-Khim 13, 119-121, 1987); enterokinase used to generate human atrial natriuretic factor (h-ANF) from chloramphenicol acetyltransferase fused h-ANF using Val-Asp-Asp-Asp-Asp-Lys as recognised amino acid sequence (A. Hobden et al., WPI 87-088 509/13); factor Xa used to generate β-globin from λC11 fused β-globin, or human calcitonin-glycine from chloramphenicol acetyltransferase fused calcitonin-glycine using (Ile/Leu/Pro/Ala)-(Glu/Asp/Gln/Asn)-Gly-Arg as a recognised amino acid sequence (Japanese Unexamined Patent Publication No. 61-135591); and V8 protease using Glu-X as a recognised amino acid sequence, factor Xa using Glu-Gly-Arg as a recognised sequence, thrombin using Arg-Ala-Leu-Leu-Ala-Gly-Pro-Arg or Gly-Pro-Arg, all used to generate atrial natriuretic peptide (ANP) from rec A protein fused ANP (Japanese Unexamined Patent Publication No. 62-135500).

A method wherein a desired protein is produced as a fused protein with a carrier protein in microbial cells has provided a powerful means of production of the desired protein. But, a method wherein a microbial cellular protein is used as a carrier protein does not provide a method of a satisfactory economical production because this method provides a lower expression level of a fused protein. Alternatively, where an exogeneous protein is used a s a carrier protein, the resultant fused protein is usually present as a soluble component in microbial cytoplasm or in peripheral space, and therefore, a complicated separation and purification process must be used to isolate the fused protein.

In additional to the above-mentioned problems, other problems arise in the fused protein approach. Namely, a desired protein itself as such produced as a fused protein is useless. Generation of a desired protein from a fused protein is necessary, and to this end, a choice of a linking peptide forming a fusion site is important. Fusion via methionine which is cleaved by cyanogen bromide is disadvantageous in that it has a limited application, and a universal linking peptide which can be cleaved by an enzyme is required. Where an amino acid sequence which can be cleaved by an enzyme (recognised amino acid sequence) is used, according to circumstances (primary amino acid sequence, secondary and tertiary structure et al.) under which a carrier protein and a desired protein are present, it may be difficult to generate a desired protein, and therefore, the extent of cleavage and proper liberation has been difficult to expect. A choice of a fused protein having a recognised amino acid sequence which site-specifically liberates a desired protein effects not only the chemical treatment or enzyme treatment for the cleavage, but also the expression, production and accumulation of the fused protein. Currently, no fused protein systems including a carrier protein and linking peptide which satisfy all of the above-mentioned requirements are known.

Human secretory leukocyte protease inhibitor (hereinafter designated as SLPI) is an inhibitory protein of an elastase derived from human polymorpho-nuclear leukocytes and present in humans mucus fluid such as parotid secretions, bronchial secretions, seminal plasma, cervial mucus, etc. An amino acid sequence of this protein isolated from a secretes from a parotid gland was determined (R.C. Thompson et al., Proc. Nat. Acad. Sci. USA 83, 6692 - 6696, 1988; PCT Japanese National Publication No. 62-501291); and a gene for this protein was isolated from a human partid gland gene library and sequenced (R. C. Thompson et al. Nucl. Acid Res. 14, 7883-7896, 1986; PCT Japanese National Publication No. 62-501262).

The SLPI inhibits a human polymorphonuclear leukocyte elastase, and therefore, is expected to be used as a therapeutic agent to arrest the emphysema, which is believed to be caused by hydrolysis of elastin by elastase. Nevertheless, the SLPI exhibits simultaneously both an elastase inhibitory activity and pancreatic trypsin inhibitory activity, and therefore, it is feared that SLPI would inhibit a physiologically important trypsin-like serine protease such as trypsin, plasmin, kallikrein, thrombin, and the like, and therefore, if the SLPI, per se, is administered, it would effect the blood coagulation-fibrinolysis system, etc., and it is difficult to use the same as a therapeutic agent for human.

EP232523 discloses the production of the C-terminal domain of the CUSI-1 protein. There is no suggestion in D1 that deletion of the N-terminal provides reduced trypsin inhibitory activity.

WO 86/03519 discloses the recombinant production of the whole SLPI protein.

Stetler et al Nucleic Acid Res. 14 (20) 7883-7896 (1986) discloses that exon III of the SLPI gene encodes the AA 57-106 domain of SLPI which corresponds to the elastase inhibitory activity.

## DISCLOSURE OF THE INVENTION

Accordingly, the present invention provides a novel elastase inhibitory polypeptide wherein elastase inhibitory activity (an inhibitory activity of chymotrypsin-like serine protease) of SLPI is maintained while an inhibitory activity of trypsin-like serine protease of SLPI is notably reduced, and a process for the production thereof, as a preconditional means therefor, an ideal carrier protein which simultaneously satisfies the requirement that a fused protein can be expressed at high level in microbial production of a desired protein by genetic engineering and the requirement that a downstream process including isolation and purification of the fused protein is easier, and a linking peptide which allows a reliable cleavage at the junction site and a release of the desired protein in an intact form.

Accordingly, the present invention generally provides an elastase inhibitory polypeptide or homopolymer thereof having an amino acid sequence represented by the formula (I):

$$X \text{---} \left( \begin{array}{l} \text{Asn-Pro-Thr-Arg-Arg-Lys-Pro-Gly-Lys-Cys-Pro-Val-} \\ \text{Thr-Tyr-Gly- Gln-Cys-Leu-Met-Leu-Asn-Pro-Pro-Asn-Phe-} \\ \text{Cys-Glu-Met-Asp-Gly-Gln-Cys-Lys-Arg-Asp-Leu-Lys-} \\ \text{Cys-Cys-Met-Gly-Met-Cys-Gly-Lys-Ser-Cys-Val-Ser-} \\ \text{Pro-Val-Lys-Ala} \end{array} \right)_n$$

$$(I)$$

or a part of the formula (I), wherein one or more than one amino acid is deleted from the N-terminal of the amino acid sequence in parentheses, wherein X represents Gly or is absent and n represents an integer of 1 to 10, characterized in that the trypsin-like serine protease inhibitory activity of the polypeptide does not exceed 1/100 of the elastase inhibitory activity.

The present invention also provides a fused protein useful as an intermediate for the production of the above-identified polypeptide represented by the formula (II'):

$$Y - B - Z_2 \quad (II')$$

wherein Y represents a carrier protein comprising a human growth hormone or a fragment thereof; $Z_2$ represents an elastase inhibitory polypeptide comprising a C-terminal portion of human secretory leukocyte protease inhibitor (SLPI) according to formula (I) and having an elastase inhibitory activity, and inhibitory activity of trypsin-like serine protease is less then or equal to 1/100 of the elastase inhibitory activity, wherein on or more than one amino acid, one or more than one amino acid is deleted; and B represents a linking peptide or homopolymer thereof having an amino acid sequence which can be chemically or biologically cleaved under the condition wherein the desired protein is not denatured; wherein B is linked to an N-terminal amino acid in $Z_2$.

The present invention moreover provides a process for the production of an elastase inhibitory polypeptide or homopolymers thereof represented by the formula (I) in claim 1 wherein X represents Gly, comprising the step of treating a fused protein represented by the formula (IV):

$$Y - (- Asn-Gly -)_n Z \qquad\qquad (IV)$$

wherein Y and Z have meanings defined under the formula (II), n represents an integer of 1 to 10, and Gly in the formula (IV) is linked to an N-terminus Asn in Z, with hydroxylamine or an analog thereof.

The present invention also provides a process for polypeptide or homopolymers thereof represented by the formula (I) in claim 1 wherein X is absent, comprising the step of treating a fused protein represented by the formula (V):

Y - B' - Z    (V)

wherein Y and Z have meanings defined under the formula (II), B' represents

$$-(-Val-Pro-Arg-)_n \quad or \quad -(-Leu-Val-Pro-Arg-)_n$$

wherein n represents an integer of 1 to 10, and Asn in X is linked to Arg in B' with thrombin or an analog thereof.

The present invention also provides a fused protein gene comprising a gene coding for human growth hormone or a fragment thereof as a carrier protein linked to a gene coding for a desired protein or a portion thereof via a gene coding for a peptide or polypeptide having an amino acid sequence which can be chemically or biologically cleaved under the condition wherein the desired protein is not denatured. As an example of the desired protein, an elastase inhibitory polypeptide having an amino acid sequence represented by the formula (III) or a portion thereof exhibiting an elastase inhibitory activity or an amino acid sequence biologically equivalent thereto is mentioned.

The present invention also provides a plasmid carrying the above-mentioned gene, and microbial cells carrying the plasmid.

BRIEF DESCRIPTION OF THE DRAWINGS

Figure 1 represents a primary amino acid sequence of SLPI, expressed by a one letter abbreviation;

Fig. 2 represents a sequence of a synthetic structural gene for $(Asn^{55}-Ala^{107})$ SLPI polypeptide using codons preferably used in E. coli, wherein $(Asn^{55}-Ala^{107})$ SLPI means a polypeptide comprising an amino acid sequence from the 55th Asn to the 107th Ala of SLPI;

Fig. 3 represents chemically synthesized oligonucleotide fragments (1) to (6) ;

Fig. 4 represents synthetic oligonucleotide fragments (7) to (10) coding for a linking peptide which links a gene coding for a $(Met^{-1}Phe^1-Phe^{139})$ human growth hormone fragment (a polypeptide comprising an amino acid sequence from the first Phe to the 139th Phe of a human growth hormone having an additional Met at N-terminus and a gene coding for $(Asn^{55}-Ala^{107})$ SLPI;

Fig. 5 represents a scheme for a construction of a plasmid for an expression of a fused protein gene (Examples 2 and 3);

Fig. 6 represents an SDS-PAGE profile of an expressed fused protein (Example 4);

Fig. 7 represents a SDS-PAGE profile obtained by thrombin treatment of the fused protein (Example 5) or by hydroxylamine treatment (Example 6);

Fig. 8 represents a result of an analysis of a mixture prepared by thrombin treatment of an S-sulfonated fused protein: The main peaks are designated as peaks 1 to 5 in the order of elution;

Fig. 9 represents SPS-PAGE profile of the peaks 1 to 5 in Fig. 8;

Fig. 10 represents a reverse HPLC elution profile of a refolded $(Asn^{55}-Ala^{107})$ SLPI prepared in Example 11;

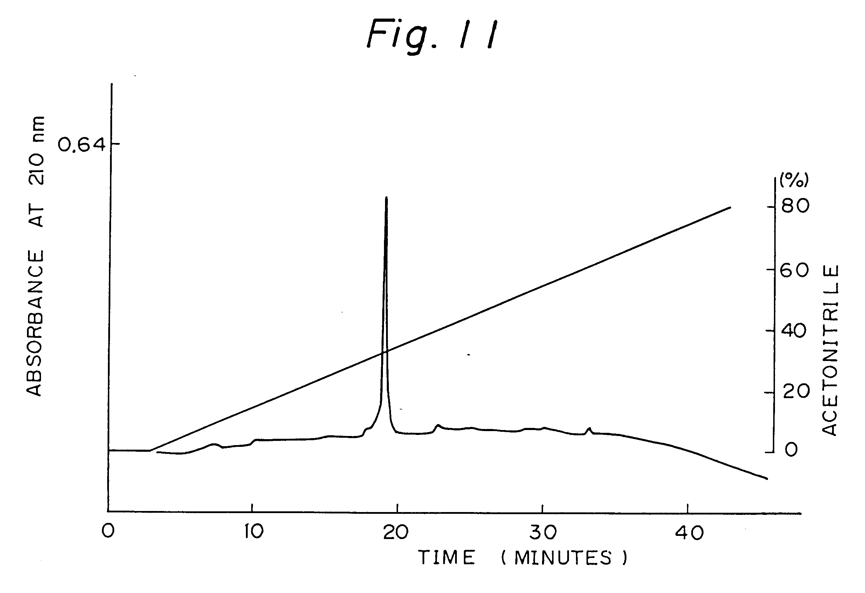

Fig. 11 represents a reverse HPLC analysis of a main peak in Fig. 10 using the same HPLC condition as shown in Fig. 10.

**BEST MODE FOR CARRYING OUT THE INVENTION**

The present inventors originally confirmed that a polypeptide comprising approximately a C-terminal half portion of SLPI starting from the first Ser and terminating at the 107th Ala has substantially no inhibitory activity of trypsin-like serine protease while maintaining an inhibitory activity of an elastase. An extent of SLPI forming the present polypeptide is not critical. Two to ten repeats of the polypeptide of the invention may form a homopolymer.

These polypeptides are represented by the formula (I):

$$X\text{-(- } Asn\text{-}Pro\text{-}Thr\text{-}Arg\text{-}Arg\text{-}Lys\text{-}Pro\text{-}Gly\text{-}Lys\text{-}Cys\text{-}Pro\text{-}Val\text{-}$$
$$Thr\text{-}Tyr\text{-}Gly\text{-}Gln\text{-}Cys\text{-}Leu\text{-}Met\text{-}Leu\text{-}Asn\text{-}Pro\text{-}Pro\text{-}Asn\text{-}Phe\text{-}$$
$$Cys\text{-}Glu\text{-}Met\text{-}Asp\text{-}Gly\text{-}Gln\text{-}Cys\text{-}Lys\text{-}Arg\text{-}Asp\text{-}Leu\text{-}Lys\text{-}Cys\text{-}$$
$$Cys\text{-}Met\text{-}Gly\text{-}Met\text{-}Cys\text{-}Gly\text{-}Lys\text{-}Ser\text{-}Cys\text{-}Val\text{-}Ser\text{-}Pro\text{-}Val\text{-}$$
$$Lys\text{-}Ala \text{ -)-} n$$

$$(I)$$

wherein X represents Gly or is absent, and n represents an integer of 1 to 10, or a part thereof exhibiting elastase inhibitory activity and trypsin-like serine protease inhibitory activity which does not exceed 1/100 of the elastase inhibitory activity or an amino acid sequence biologically equivalent thereto.

According to the methods used for cleavage of a fused protein in the production of the present polypeptide, an amino-terminus of the polypeptide is either Asn wherein X is absent of Gly attached to Asn wherein X represents Gly.

Note, the present invention includes, in addition to the polypeptide having the above-mentioned amino acid sequence, polypeptides having an amino acid sequence wherein one or more than one amino acid is deleted, to the extent that they exhibit the above-mentioned biological activity.

These proteins cannot be produced by a conventional procedure such as a treatment of SLPI with a proteolytic enzyme, but produced only by a gene expression in microbial cells by a recombinant gene technology of the present invention. Namely, since the desired protein is a low molecular weight protein, it can be produced only by the present process wherein the desired protein is genetically produced in a form of a fused protein containing, for example, a human growth hormone as a carrier protein, and the fused protein is cleaved with an enzyme such as thrombin or chemicals such as hydroxylamine to generate the desired protein.

Accordingly, the present invention provides a fused protein represented by the above-mentioned formula (II') useful as an intermediate for the production of the above-mentioned elastase inhibitory polypeptide. Within the fused protein represented by the formula (II'), a fused protein wherein the desired protein comprises the 55th Asn to the 107th Ala of SLPI is represented by the following formula (II):

Y - B - Z    (II)

wherein Y represents a carrier protein comprising a human growth hormone or fragment thereof;

Z represents a polypeptide which is an elastase inhibitory polypeptide or homopolymers thereof having a sequence represented by the formula (III):

$$( \ Asn\text{-}Pro\text{-}Thr\text{-}Arg\text{-}Arg\text{-}Lys\text{-}Pro\text{-}Gly\text{-}Lys\text{-}Cys\text{-}Pro\text{-}Val\text{-}$$
$$Thr\text{-}Tyr\text{-}Gly\text{-}Gln\text{-}Cys\text{-}Leu\text{-}Met\text{-}Leu\text{-}Asn\text{-}$$
$$Pro\text{-}Pro\text{-}Asn\text{-}Phe\text{-}Cys\text{-}Glu\text{-}Met\text{-}Asp\text{-}Gly\text{-}Gln\text{-}Cys\text{-}Lys\text{-}$$
$$Arg\text{-}Asp\text{-}Leu\text{-}Lys\text{-}Cys\text{-}Cys\text{-}Met\text{-}Gly\text{-}Met\text{-}Cys\text{-}Gly\text{-}Lys\text{-}$$
$$Ser\text{-}Cys\text{-}Val\text{-}Ser\text{-}Pro\text{-}Val\text{-}Lys\text{-}Ala \ )_{n}$$

$$(III)$$

wherein n represents an integer of 1 to 10, or a portion thereof exhibiting an inhibitory activity of elastase or an amino acid sequence biologically equivalent thereto; and

B represents a linking peptide or homopolymer thereof having an amino acid sequence which can be chemically or biologically cleaved under the condition wherein the desired protein is not denatured; wherein B is joined to an amino terminus, Asn in Z.

The carrier protein in the formula (II') and (II) is preferably a human growth hormone or a portion thereof which provides a higher expression level of fused protein and is present as inclusion bodies in microbial cells, and is easy to isolate and purify. The human growth hormone is a polypeptide consisting of 191 amino acid residues starting with the first Phe and terminating at the 191th Phe. Where a desired gene is expressed using a carrier protein in E. coli, since Met residue is essential for the initiation codon of translation, an expressed protein must be a methionyl human growth hormone. Therefore, such a protein also fall within the scope of the present carrier protein. Moreover, modified human growth hormones wherein the amino acid sequence of a native growth hormone is partially changed, fall within the scope of the present carrier protein. An example of such a modified human growth hormone is that wherein the 53th cysteine is replaced by another amino acid. A preferable portion of a human growth hormone used as a carrier protein is a fragment from the first Phe to the 139th Phe, or a fragment from the first Phe to the 122th Gln.

A linking peptide or homopolymer thereof at a judion site includes any recognized amino acid sequence including known sequences, which can be chemically or biologically cleaved under the condition wherein the desired protein is not denatured.

For example Asn-Gly (P. Bornstein, Meth. Enzymol. 47, 132, 1977) or a repeating sequence thereof ( Asn-Gly )$_n$ wherein n represents an integer of 1 to 10 can be used as a linking peptide which can be cleaved by hydroxylamine to generate the desired protein. Moreover, as a fusion amino acid sequence which can be cleaved by thrombin, although a reported amino acid sequence

**Ala-Asp-Ser-Gly-Glu-Gly-Asp-Phe-Leu-Ala-Glu-Gly-Gly-Gly-Val-Arg-Glu-Gly-Val-Asn-Asp-Asn-Glu-Glu-Gly-Phe-Phe-Ser-Ala-Arg, or Asp-Asp-Pro-Pro-Thr-Val-Glu-Leu-Gln-Cly-Leu-Val-Pro-Arg**

(B. Blomback et al., BBA 115, 371 - 396, 1966; T. Takagi et al., Biochemistry 13, 750 - 756, 1974) can be used, a shorter amino acid sequence Val-Pro-Arg or Leu-Val-Pro-Arg, or repeating sequences thereof ( Val-Pro-Arg )$_n$ or ( Leu-Val-Pro-Arg )$_n$ wherein n represents an integer of 1 to 10 is preferably used to reliably release and separate a desired protein without affect on a primary amino acid sequence and steric effect of the carrier protein and desired protein. In these cases, a C-terminus Gly, Arg or Arg in B is joined to an N-terminus, Asn in Z respectively.

To produce the present polypeptide represented by the formula (I), a fused protein represented by the formula (II) is cleaved at its junction site of B. Where this cleavage should be carried out by hydroxylamine or an analog thereof, ( Asn-Gly )$_n$ is used as B. Namely, a fused protein represented by the above-mentioned formula (IV) is treated with hydroxylamine or an analog thereof such as alkyl hydroxylamine, hydrazine or the like to obtain an elastase inhibitory polypeptide or homopolymer thereof wherein X in the formula (I) is Gly. Alternatively, where cleavage at a linking peptide B should be carried out by thrombin or an analog thereof, for example, ( Val-Pro-Arg )$_n$ or ( Leu-Val-Pro-Arg )$_n$ is used as B. Namely, a fused protein represented by the above-mentioned formula (V) is treated with a thrombin or analog thereof such as human thrombin, bobine thrombin, equine thrombin, porcine thrombin, or the like to obtain an elastase inhibitory polypeptide or homopolymer thereof represented by the formula (I) wherein X is absent.

A fused protein represented by the formula (II) is produced according to a recombinant gene technology known per se. In this case, the present invention is characterized by using a fused protein gene comprising a gene coding for a human growth hormone or a fragment thereof as a carrier protein linked to a gene coding for a desired protein or a portion thereof via a gene coding for a peptide or polypeptide having an amino acid sequence which can be chemically or biologically cleaved under the condition wherein the desired protein is not denatured.

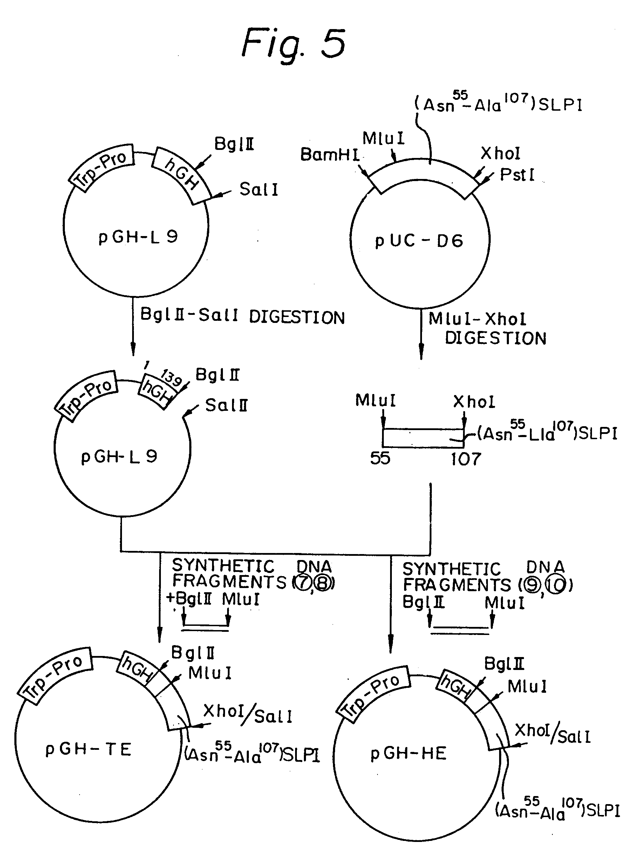

A process for the construction of plasmids pGH-TE and pGH-HE, which express a fused protein comprising (Met$^{-1}$Phe$^1$-Phe$^{139}$) human growth hormone fragment and (Asn$^{55}$-Ala$^{107}$) SLPI fragment polypeptide, is shown in Fig. 5.

pGH-L9, an expression plasmid of human growth hormone (Proc. Natl. Acad. Sci. USA., 81 5956, 1984) is digested with restriction enzymes BglII and SalI to eliminate a 1/3 downstream part of the human growth hormone gene. On the other hand, a plasmid pUC-D6 comprising ($Asn^{55}$-$Ala^{107}$) SLPI gene is digested with MluI and XhoI to obtain an ($Asn^{55}$-$Ala^{107}$) SLPI gene fragment.

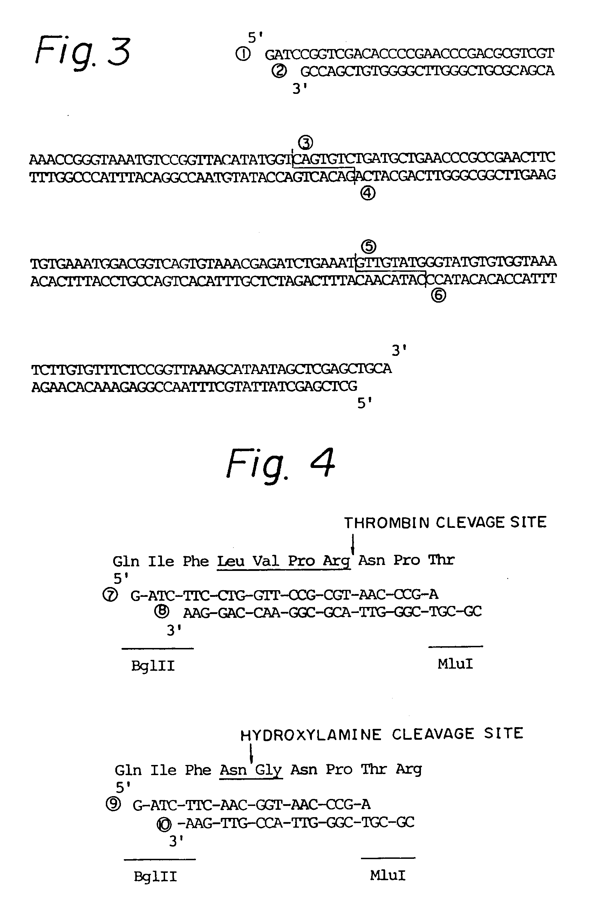

The above-mentioned two fragments, and synthetic DNA linkers (7) and (8), or (9) and (10), shown in Fig. 4, are ligated using T4 DNA ligase to obtain plasmids pGH-TE and pGH-HE expressing fusion proteins comprising a growth hormone fragment and ($Asn^{55}$-$Ala^{107}$) SLPI fragment. In Fig. 5, pGH-TE is a plasmid expressing a fused protein containing a thrombin-cleaved sequence, and pGH-HE is a plasmid expressing a fused protein containing a hydroxylamine-cleaved-sequence.

A gene coding for a recognized amino acid sequence which links a gene coding for a desired protein and a gene coding for a human growth hormone or a portion thereof may be any Generated gene in a same reading frame with the human growth hormone gene or a portion thereof coding for an amino acid sequence, preferably ( Asn-Gly )$_n$ wherein n represents an integer of 1 to 10, which can be easily cleaved by a hydroxylamine treatment to generate a desired protein. Alternatively, it may be any degenerated gene coding for an amino acid sequence, preferably ( Val-Pro-Arg )$_n$ or ( Leu-Val-Pro-Arg )$_n$ wherein n represents an integer of 1 to 10, which can be easily cleaved by a thrombin treatment to generate a desired protein.

To ally a same reading frame between a gene coding for a carrier protein, i.e., human growth hormone or a portion thereof and a recognized amino acid sequence, a synthetic DNA linker can be inserted therebetween.

Genes coding for desired proteins include those genes which code for hormones or factors such as somatomedin, IGF-I, IGF-II, EGF (epidermal growth factor), PDGF (platelet-derived growth factor). Moreover, they are genes coding for lymphokines, enzymes, enzyme inhibitory proteins. For example, interferons, interleukins, neuropeptides, intestinal peptides, blood coagulation factors such as Factor VII, Factor VIIIC, Factor IX, Protein C and Protein S, $\alpha_1$-antitrypsin, SLPI, TIMP (tissue inhibitor of metalloproteinase, and proteins of lumg surfactant. Moreover, gene coding for a desired protein include genes coding for an antibody or a portion thereof, and complement or a portion thereof.

Desired proteins may be not only native proteins, but also modified proteins or fragments thereof, and in the latter cases, a gene capable of expressing the modified proteins or fragments thereof may be used.

According to the present invention, to obtain an elastase inhibitory polypeptide or homopolymers thereof, a gene coding for a carboxy-terminal half of SLPI or a gene comprising a two to ten-times repeat of the above-mentioned gene may be used.

A fused protein gene of the present invention may be introduced in to an expression plasmid by joining an appropriate promoter downstream thereof. Available promoters include a tryptophan operon promoter (trp promoter), lactose operon promoter (lac promoter), tac promoter, PL promoter, lpp promoter, and the like. Especially, a 5'-flanking sequence of pGH-L9 wherein a trp promoter and a optimized space between an SD sequence and a translation initiation codon ATG are used, is preferable (Japanese Unexamined Patent Publication No. 60-234584).

For an efficient expression of a fused protein, a trp promoter, SD sequence, translation-initiation codon, gene coding for a human growth hormone or a portion thereof, a gene coding for a linking peptide, a gene coding for a desired protein or a portion thereof, and a translation termination codon, are allied in this order. Such allied genes are inserted into an appropriate plasmid such as pBR322 or related plasmid to construct a expression plasmid of a fused protein. Note, a plasmid pBR 322 is preferably used.

Microbial host cells for an expression of the present fused protein gene include E. coli, Bacillus subtilis, and the like, and E. coli especially preferable. The above-mentioned expression plasmid of a fused protein can be introduced into a microbial cell such as an E. coli cell, by a known method, M.V. Norgard et al., Gene, 3, 279, 1978.

The transformed microorganism thus obtained is cultured in accordance with a method known per se. As a medium, an M9 medium (T, Maniatis ed. Molecular Cloning, p 440, Spring Harbor Laboratory, New York, 1982) containing glucose and casamino acid is mentioned, and if it is necessary to stabilize an expression plasmid in host cells, ampicillin, etc., can be added.

Cell culturing is carried out under a condition suitable for a transformed microorganism, for example, with aeration and agitation by shaking at 37°C for 2 to 36 hours. Moreover, to stimulate an efficient action of a promoter, an inducing agent such as 3-$\beta$-indole acrylic acid (when a trp promoter is used), and isopropyl-$\beta$-D-thiogalactoside (when a tac promoter is used), etc., can be added.

After culturing, the transformed microbial cells are collected, for example, by centrifugation, resuspended, for example, in a phosphate buffer, disrupted, for example, by ultrasonication, and centrifugated to easily obtain a fused protein in a pure form. An advantage of the present invention is that the fused protein as such can be cleaved by hydroxylamine or thrombin treatment to generate a desired protein. If necessary, cysteine residues of the fused protein can be sulfonated, followed by treatment with thrombin to generate a sulfonated molecule of the desired protein.

A fused protein joined via an amino acid sequence which can be cleaved by hydroxylamine (including analogs thereof, i.e., compounds which have a structure similar to and an action the same as hydroxylamine) is treated with hydroxylamine under an alkaline condition at 45°C for two to four hours, or a fused protein joined via an amino acid sequence which can be cleaved by thrombin (including analogs thereof, i.e., enzymes which have a structure similar to and a biological action the same as thrombin) is treated with thrombin at 37°C, for 2 to 24 hours, to generate a desired protein from the fused protein, and isolate the same.

If a desired protein has tertiary structure via disulfide bonds, it can be converted to a biologically active molecule having the same tartiary structure as a native protein by, for example, a procedure of Chance et al. (R.E. Chance et al., Peptides: Seventh U.S. Peptide Symposium Proceedings, DH. Rich and E. Gross ed., 721 - 728, Pierce Chemical Co., Rockford, IL., 1981). In a process involving sulfonation, a sulfonated desired protein is reduced, followed by intramolecular disulfide bond formation to prepare a biologically active protein having the same tertiary structure as a native protein.

The (Asn$^{55}$-Ala$^{107}$) SLPI polypeptide of the present invention, i.e., an elastase inhibitory polypeptide, can be isolated and purified from a reaction mixture by an appropriate combination of isolation and purification procedures known per se. These known isolation and purification procedures include methods of using a difference of solubility such as salting out and solvent precipitation; methods mainly using a difference of molecular weight such as dialysis, ultrafiltration, gel filtration, and SDS-polyacrylamide gel electrophoresis; methods using a difference of electric charge such as ion-exchange chromatography, and ion-exchange high performance liquid chromatography; methods using a specific affinity such as affinity chromatography; methods using a difference of hydrophobicity such as a reverse high performance liquid chromatography; and methods using a difference of an isoelectric point such as electrofocusing.

As shown in Table 4, in a comparison of the inhibitory activities of the present elastase inhibitory polypeptide to various proteolytic enzymes, although an elastase inhibitory activity of SLPI is maintained, an inhibitory activities of trypsin-like serine protease such as human thrombin, human plasmin, and human kallikrein are remarkably reduced. Therefore, in comparison to SLPI, an inhibitory activity of elastase over trypsin of the present (Asn$^{55}$-Ala$^{107}$)SLPI polypeptide is improved, so the present polypeptide is promising as a useful clinical medicament.

Since the present protein has an excellent inhibitory activity of a polymorphonuclear leukocyte elastase, it may be used as a therapeutic agent for various diseases involving leukocyte elastase-mediated tissue destruction, such as emphysema, rheumatoid arthritis, glomerulo nephritis, periodontal disease, and amyotrophia, etc., as well as ARDS, neutrocytic allergic lung disease involved in neutrophile, and septicemia.

The present protein, if necessary, can be lyophilized to form a powder. In this lyophilization, a stabilizer such as sorbitol, mannitol, dextrose, maltose, glycerol, human serum albumin, or the like is used.

The present protein is used for the treatment of the above-identified diseases in the form of a pharmacologically acceptable formulation. The administration routes are oral, or parenteral, for example, intravenus, intramuscular, subcutaneous, percutaneous, rectal, and perrespiratory truck (intratrachial, intranasal) administrations, as well as an administration using an ionophoretic device.

Note, in the specification and the drawings, where amino acids and peptides, etc., are described by abbreviations, the symbols used are those according to IUPAC-IUB (Commission on Biological Nomenclature) or symbols conventionally used in the art.

## Examples

Next, the present invention is described in more detail by way of Examples, but the invention is not limited to these Examples.

Note, the various genetic engineering techniques used in the Examples are as follows.

## (1) Cleavage of DNA with restriction enzyme (Method 1)

DNA in an amount of 0.1 to 1 μg was dissolved in 10 μl of a restriction enzyme buffer (for MluI or PstI cleavage, 10 mM Tris-HCl, pH 7.5, 10 mM MgCl, 0.1 mg/ml gelatin, 60 mM NaCl, 6 mM mercaptoethanol; for BamHI, BglII, NdeI, SalI, or XhoI cleavage, 10 mM Tris-HCl, pH 7.5, 10 mM MgCl$_2$ , 0.1 mg/ml gelatin, 150 mM NaCl and 6 mM mercaptoethanols, all in a final concentration in an aqueous solution), 2 to 4 units of the restriction enzyme was added to the solution, and a reaction was carried out at 37°C for one hour.

(2) Agarose gel electrophoresis (Method 2)

After the restriction enzyme cleavage, 3 $\mu$l of a solution of 0.25% bromophenol blue in 50% aqueous glycerol was added, and an agarose gel electrophoresis (concentration 0.7 to 1%) was carried out.

As an electrophoresis buffer, 90 mM Tris-borate (pH 8.0), 2 mM EDTA aqueous solution was used.

(3) Recovery of DNA fragment from agarose gel (Method 3)

Agarose gel electrophoresis was carried out using a low melting point agarose gel, a band corresponding to a desired DNA was cut out, and the DNA was recovered by a method of L. Weislander et al., Anol. Biochem., 98, 305 (1978).

(4) Ligation using T4 DNA ligase (Method 4)

DNA fragments to be ligated were mixed, and after ethanol co-precipitation, the preciptitate was dissolved in 20 $\mu$l of a ligation buffer (66 mM Tris-HCl, pH 7.6, 6.6 mM $MgCl_2$ , 10 mM dithiothreitol, 1 mM ATP), and 2 to 10 units of T4 DNA ligase was added to the solution, and a reaction was carried out at 16°C for 12 hours.

(5) Preparation of competent cells and transformation (Method 5)

E. coli was transformed according to a modification of a standard $CaCl_2$ method (M.V. Norgard et al). Namely, An 18-hour culture of E. coli HB101 was inoculated to 5 ml of L medium (1% trypton, 0.5% yeast extract, 0.5% NaCl, pH 7.2), and grown to a turbidity at 600 nm ($OD_{600}$) of 0.3. The cells were twice washed in a cold magnesium buffer (0.1 M NaCl, 5 mM $MgCl_2$ , 5 mM Tris-HCl, pH 7.6, 4°C), and resuspended in 2 ml of a cold calcium buffer (100 mM $CaCl_2$ , 250 mM KCl, 5 mM $MgCl_2$ , 5 mM Tris-HCl, pH 7.6, 4°C), and the suspension was allowed to stand at 4°C for 25 minutes. After collection, the cells were suspended in 200 $\mu$l of a cold calcium buffer, and the cell suspension was mixed with the solution of the ligation reaction at a ratio of 10:1 (v/v). This mixture was maintained at 4°C for 60 minutes, 1 ml of an LBG medium (1% trypton, 0.5% yeast extract, 1% NaCl, 0.08% glucose, pH 7.2) was added to the mixture, and cell culturing was carried out at 37°C for one hour. The culture broth was inoculated to a selection medium (L medium plate containing 30 $\mu$g/ml ampicillin) at a ratio of 100 $\mu$l/plate. The plate was incubated at 37°C overnight to grow transformants.

A plasmid DNA was prepared from the resultant ampicillin resistant colonies by a method of Birnboim, H.C. and J. Doly Nucleic Acids Res., 7, 1513 (1979), and digested with appropriate restriction enzymes. The digestion pattern was analyzed by agarose gel electrophoresis to obtain a desired clone.

(6) Determination of DNA nucleotide sequence (Method 6)

DNA sequencing was carried out by a method of Chen, E.Y. and Seeburg, P.H., DNA, 4, 165 (1985), using the plasmid DNA as a template and M13 primer M3, RV or pBR322 primer S2 (both from Takara Shuzo, Japan) as a primer, and an M13 sequence kit (Amersham Japan).

(7) Other procedures

All other DNA manipulations were carried out by the methods of Maniatis et al., Molecular Clonings, Cold Spring Harbor Laboratory, New York, 1982.

Example 1. Synthesis and subcloning of structure gene for (Asn[55]-Ala[107]) SLPI polypeptide fragment

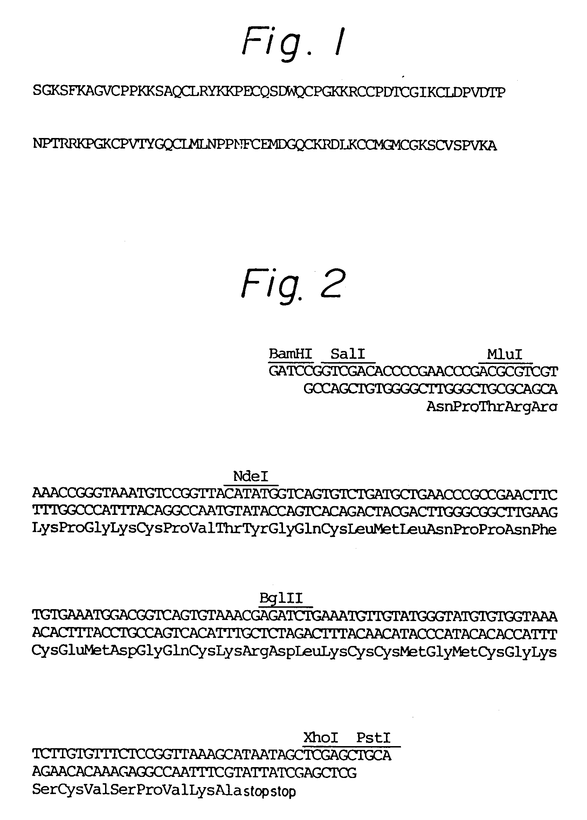

The gene for the (Asn[55]-Ala[107]) SLPI fragment was designed on the basis of an amino acid sequence of SLPI as shown in Fig. 1 (R.C. Thompson et al., Proc. Natl. Acad. Sci. USA, 83, 6692, 1986; V. Seemuller et al., FEBS Lett., 199, 43, 1986) by choosing codons frequently used in E. coli and providing restriction enzyme recognizing sites at appropriate positions as shown in Fig. 2 for the construction of a desired gene. Next, the designed nucleotide sequence was divided into 3 portions as shown in Fig. 3 to synthesize six oligonucleotides. The oligonucleotides were synthesized using a full automatic DNA synthesizer (Applied Biosystems, Model 381A), by the phosphoamidite method. Synthetic oligonucleotides were purified according to the Applied Biosystems protocol. Namely, synthetic oligonucleotide in an aqueous ammonia was maintained at 55°C overnight to deprotect amino radicals of bases of DNA, and a higher molecular weight synthetic oligonucleotide fraction was separated by gel filtration using Sephadex G-50 fine gel (Pharmacia). Next, the oligonucleotide fraction was subjected to polyacrylamide electrophoresis (gel concentration 20%, containing 7 M urea), and a migration pattern was observed by ultra-violet shadowing. A band corresponding to a desired origonucleotide was cut out and cut into small pieces, and two to five ml of a DNA eluting buffer (500 mM $NH_4OAc$, 1 mM EDTA, 0.1% SDS, pH 7.5) was added to the gel pieces, and the whole was shaken at 37°C overnight. An aqueous solution containing the desired oligonucleotide was taken by centrifugation. Finally, the solution containing a synthetic oligonucleotide was applied to a gel filtration column (Sephadex G-50) to obtain a purified synthetic oligonucleotide preparation. Note, if necessary, the polyacrylamide gel electrophoresis was repeated to improve the purity of the synthetic oligonucleotide. The synthetic oligonucleotide in an amount of 0.1 to 1.0 $\mu$g was subjected to polynucleotide kinase reaction in the presence of 1 mM ATP, to phosphorylate its 5'-terminal.

The phosphorylation was carried out in a 50 mM Tris-HCl (pH 9.5), 10 mM $MgCl_2$, 5 mM dithiothreitol aqueous solution using 5 units of polynucleotide kinase (P-L Biochemicals). Two synthetic oligonucleotides phosphorylated at the 5'-terminal thereof corresponding to the upper chain and lower chain shown in Fig. 3 were taken into aqueous solution, mixed, and were gradually cooled from 70°C to room temperature, to anneal the two oligonucleotides.

For subcloning, 1 $\mu$g of plasmid pUC119 (Takara Shuzo, Japan) was cleaved with BamHI and PstI by the method 1, the linearized fragments were separated by agarose gel electrophoresis by the method 2, and the linear plasmid was recovered by the method 3. The recovered linear plasmid was mixed with 6 $\mu$g of the annealed synthetic fragments (1) and (2), (3) and (4), or (5) and (6), and after ethanol precipitation, were ligated, using a T4 DNA ligase, by the method 4.

The ligation mixture was added to 200 $\mu$l of the competent cells of E. coli prepared by the method 5, and transformation was carried out by the method 5. From colonies grown on a selective culture medium (method 5), plasmid DNA was prepared by the method 5. The construction of a desired subclone pUC-D6 containing a carboxy terminal half of SLPI, $Asn^{55}$-$Ala^{107}$, was confirmed by cleaving with a restriction enzyme BamHI, SalI, MluI, NdeI, XhoI or PstI by the method 1, and observing cleavage patterns by agarose gel electrophoresis, by the method 2. Also, the DNA was sequenced by the method 6 to confirm a nucleotide sequence of the subclone.

Example 2. Preparation of plasmid for expression of a fused protein comprising ($Met^{-1}Phe^{1}$-$Phe^{139}$) human growth hormone fragment and ($Asn^{55}$-$Ala^{107}$) SLPI fragment polypeptide linked via a thrombin cleaved site, and transformant thereof

As shown in Fig. 5, 1 $\mu$g of an expression plasmid for an expression of human growth hormone gene (M. Ikehara et al., Proc. Natl. Acad. Sci. USA, 81, 5956, 1984) was cleaved with BglII and SalI, and the resulting fragments were separated by agarose electrophoresis by the method 2, and recovered from the gel by the method 3.

Moreover, 2 $\mu$g of pUC-D6 obtained in Example 1 was cleaved with MluI and XhoI, the resulting fragments were separated, and a DNA fragment of about 0.15 Kbp was recovered by the method 3. On the other hand, DNA fragments (7) and (8) shown in Fig. 4 coding for an amino acid sequence which can be cleaved by thrombin were chemically synthesized. Next, 1 $\mu$g of each of the two DNA fragments recovered as described above, and the annealed synthetic fragments (7) and (8), were mixed, and after an ethanol precipitation, ligation was carried out using a T4 DNA ligase by the method 4, E. coli HB101 was transformed with the ligation mixture by the same procedure as in the Example 1, and from colonies grown on a selective medium, a transformant carrying an expression plasmid pGH-TE of the desired fused protein gene was obtained. The construct of the plasmid pGH-TE was confirmed by the same procedure as in Example 1.

Note, E. coli HB101 containing the plasmid pGH-TE was designated as Escherichia coli HB101 (pGH-TE), and deposited with the Fermentation Research Institute, Agency of Industrial Science and Technology (FRI), Higashi 1-1-3, Tsukuba-shi, Ibaraki-ken, Japan as an international deposition under the Budapest Treaty, as FERM BP-2168 on December 1, 1988.

Example 3. Preparation of plasmid for expression of a fused protein comprising ($Met^{-1}Phe^{1}$-$Phe^{139}$) human growth hormone peptide and ($Asn^{55}$-$Ala^{107}$) SLPI fragment polypeptide linked via a hydroxylamine cleaved site and transformant thereof.

As shown in Fig. 5, the same procedure as described in Example 2 was repeated except that synthetic DNA fragments (9) and (10) shown in Fig. 4 coding an amino acid sequence which can be cleaved by hydroxylamine instead of the synthetic DNA fragments (7) and (8) were used to obtain a fused protein expression plasmid pGH-HE, and an E. coli HB101 transformant carrying the plasmid.

Note, E. coli HB101 containing the plasmid pGH-HE was designated as Escherichia coli HB101 (pGH-HE), and deposited with the FRI as an International Deposition under the Budapest Treaty, as FERM BP-2167 on December 1, 1988.

Example 4. Expression of a fused protein gene

E. coli HB101 carrying the fused protein gene expression plasmid pGH-TE and E. coli HB101 carrying the fused protein gene expression plasmid pGH-HE, prepared in Examples 2 and 3, respectively, were separately inoculated in an L medium (1% trypton, 0.5% yeast extract, 1% NaCl, pH 7.5, autoclaved) containing 50 to 100 $\mu$g/ml ampicillin, and cultured overnight.

M9 medium (0.6% $Na_2HPO_4$ , 0.3% $KH_2PO_4$ , 0.05% NaCl, 0.1% $NH_4Cl$ aqueous solution, pH 7.4) containing 0.2% glucose and 5 mg/ml casamino acid was autoclaved, and to the medium were added a separately autoclaved $MgSO_4$ aqueous solution and $CaCl_2$ aqueous solution, to a final concentration of 2 mM and 0.1 mM respectively. To this medium was added the above-mentioned over night cell culture to 0.1 of $OD_{660}$ , and culturing was carried out at 37°C. When the $OD_{660}$ reached 0.5, 3-$\beta$-indoleacrylicacid was added to the culture to a final concentration of 40 $\mu$g/ml, and culturing was continued with shaking until the $OD_{660}$ reached 1.0 at 37°C. Then, the cells were collected by centrifugation, and washed with a TE buffer (50 mM Tris-HCl, 4 mM EDTA, pH 7.5).

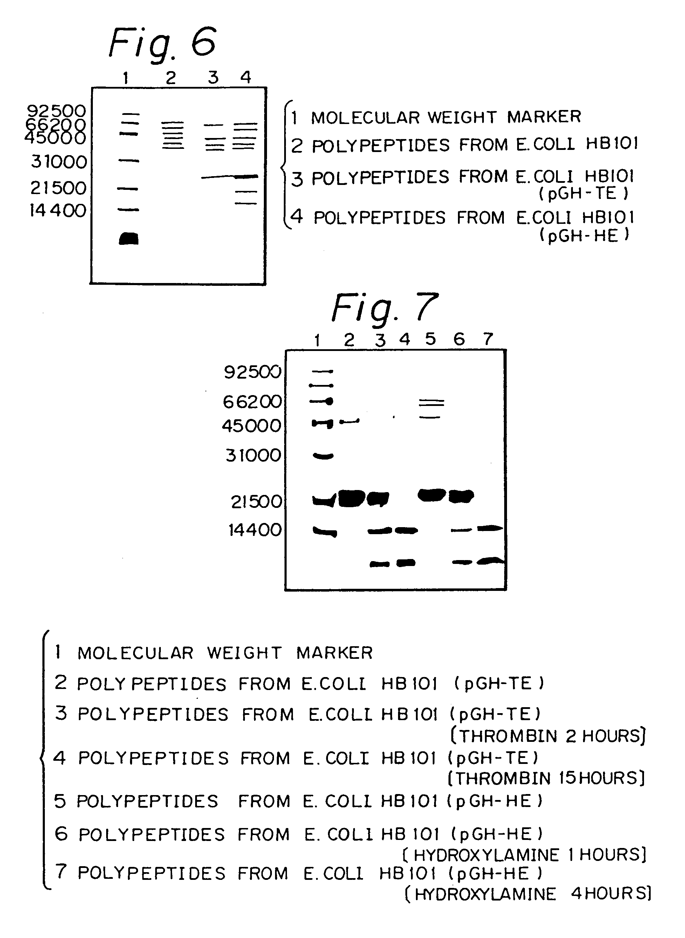

The washed cells were suspended in a 1/10 volume of the TE buffer and disrupted with an ultrasonicator (Kubota Shoji, Type 200M). The disruptant was centrifuged to obtain a precipitate containing a desired fused protein in the form of inclusion bodies. The precipitate was washed with a 0.5% Triton X-100, 1 mM EDTA aqueous solution, washed again with a TE buffer, and dissolved in a 7 M urea, 20 mM Tris-HCl (pH 8.0) aqueous solution or 6 M guanidine hydrochloride, 20 mM Tris-HCl (pH 8.0). The solution was dialyzed against 20 mM Tris-Hl (pH 8.0) to obtain a fused protein aqueous solutions and to the resulting aqueous solution were added a Tris-HCl buffer (pH 6.8), SDS, and 2-mercaptoethanol, ethanol, and glycerol to final concentrations of 60 mM, 2%, 4%, and 10%, respectively, and the mixture was subjected to SDS-polyacrylamide gel electrophoresis (O.K. Laemmli, Nature, 227, 650 (1970). The results are shown in Fig. 6. In Fig. 6, lane 1 represents the following molecular weight size maker, lane 2 represents proteins derived from E. coli HB101, lane 3 represents proteins obtained from E. coli HB101/pGH-TE, and lane 4 represents proteins obtained from E. coli HB101 (pGH-HE), all showing electrophoresis profiles.

Note, subsequently the fused proteins were purified by ion exchange chromatography and reverse chromatography.

| Protein | Molecular Weight |
|---|---|
| Lysozyme | 14,400 |
| Soybean trypsin inhibitor | 21,500 |
| Carbonic anhydrase | 31,000 |
| Ovalbumin | 45,000 |
| Bovine serum albumin | 66,200 |
| Phosphorylase B | 92,500 |

Example 5. Cleavage of fused protein with thrombin

To the protein solution obtained in Example 4 by culturing E. coli HB101 containing pGH-TH, thrombin (Sigma) was added in an amount of 1/200 by weight relative to the total protein, and a reaction was carried out at 37°C for 15 hours. The reaction mixture was treated by the same procedure as in Example 4, and subjected to SDS-polyacrylamide gel electrophoresis. The results are shown in Fig. 7, in lanes 2 to 4. It was confirmed that a fused protein having a molecular weight of about 20,000 was cleaved with thrombin showing two bands corresponding to a peptide having a molecular weight of about 14,000 derived from a human growth hormone and a desired ($Asn^{55}$-$Ala^{107}$) SLPI fragment polypeptide having a molecular weight of about 6,000. Note, in Fig. 7, lane 1 represents the same molecular weight makers as in Fig. 6.

13

Example 6. Cleavage of fused protein with hydroxylamine

The protein solution obtained in Example 4 by culturing E. coli HB101 containing pGH-HE was adjusted to final concentrations of 2 M hydroxylamine and 0.2 M Tris (pH 9.0), and a reaction was carried out at 45°C for 4 hours. The reaction mixture was treated by the same procedure as in Example 4, and subjected to SDS-polyacrylamide gel electrophoresis. The results are shown in Fig. 7, in lanes 5 to 7. A fusion protein having a molecular weight of about 20,000 was cleaved with hydroxylamine showing two bands corresponding to a polypeptide, having a molecular weight of about 14,000, derived from a human growth hormone and a desired N-terminal glycyl ($Asn^{55}$-$Ala^{107}$) SLPI fragment polypeptide having a molecular weight of about 6,000.

Example 7.

From the SDS-polyacrylamide gel obtained in Examples 5 and 6, a band containing the desired SLPI polypeptide fragment was cut out, the polypeptide was extracted with 70% formic acid from the gel, and the extract was filtered, and after filtration, the filtrate was dried under a reduced pressure. The dried sample was dissolved in trifruoloacetic acid, immobilized on a polybrene coated filter according to the protocol of Applied Biosystems, and an amino acid sequence from the N-terminal was determined by cleaving PTH-amino acids from the N-terminal by a protein sequencer (Applied Biosystems 740A) and analyzing the result by a PTH analyzer (Applied Biosystems 120A). The results of the amino acid sequence from the N-terminal are shown in Table 1.

Table 1

| Cycle No. | ($Asn^{55}$-$Ala^{107}$) SLPI fragment | Experimental result | |
|---|---|---|---|
| | | Thrombin treatment | Hydroxylamine treatment |
| 1 | Asn | Asn | Gly |
| 2 | Pro | Pro | Asn |
| 3 | Thr | Thr | Pro |
| 4 | Arg | Arg | Thr |
| 5 | Arg | Arg | Arg |

From these results, it is confirmed that an amino acid sequence at an N-terminal of each polypeptide obtained as above conformed to an amino acid sequence of the desired ($Asn^{55}$-$Ala^{107}$) SLPI fragment. That is, it was confirmed that a ($Asn^{55}$-$Ala^{107}$) SLPI fragment polypeptide was correctly liberated by thrombin or hydroxylamine.

Example 8.

The elastase inhibitory activities of the above-mentioned expressed fused protein, thrombin-cleaved product, and hydroxylamine-cleaved product were evaluated as follows.

Reagent solution

Buffer: 0.1 M HEPES, 1.0 M NaCl, 0.1% PEG-600 (pH 7.5)
Elastase (polymorphonuclear leukocyte elastase from human sputum): elastase (EPC Co., Funakoshi Yakuhin) 2 mg/ml of elastase in the buffer (stock solution) was diluted 30,000-fold in said buffer ($1.0 \times 10^{-8}$ M).
Substrate solution: 18 mg/ml of MeO-Suc-Ala-Ala-Pro-Val-pNA (Backem) in DMSO (stock solution) was diluted 10-fold in said buffer ($3 \times 10^{-3}$ M).
To each well of a 96-well ELISA microplate, were added 140 $\mu$l of the above-mentioned buffer, 20 $\mu$l of the test sample solution, and 20 $\mu$l of the elastase solution. This mixture was stirred at 37°C for one hour, 20 $\mu$l of the substrate solution was added to each well, and a reaction was carried out at 37°C for one hour to develop same. The absorption at 405 nm was measured and the results are shown in Table 2.

14

Table 2

| Sample | (treatment) | Elastase inhibitory activity |
|---|---|---|
| pGH-L9/HB101 | (-) | - |
| | (+) | - |
| pGH-TE/HB101 | (-) | + |
| | (+) | + |
| pGH-HE/HB101 | (-) | + |
| | (+) | + |

Fusion protein exhibited an elastase inhibitory activity regardless of the thrombin treatment or hydroxylamine treatment.

Example 9. Sulfonation of cysterine residue of fused protein

250 mg of the fused protein obtained in Example 5 was dissolved in 100 ml of 7 M urea, 0.5 M Tris-HCl (pH 8.2), and sodium sulfite (Wako Junyaku) was added to the resulting solution to a final concentration of 0.3 mM, and reacted at 45°C for 30 minutes. Next, sodium tetrathionate (Sigma) was added to a final concentration of 0.05 mM to react at 45°C for 30 minutes. The reaction mixture was put into a dialysis tube (10 K) and dialyzed once against 10 ℓ of water, and twice against 10 ℓ of a 50 mM Tris-HCl buffer (pH 8.5).

Example 10. Cleavage of sulfonated fused protein with thrombin

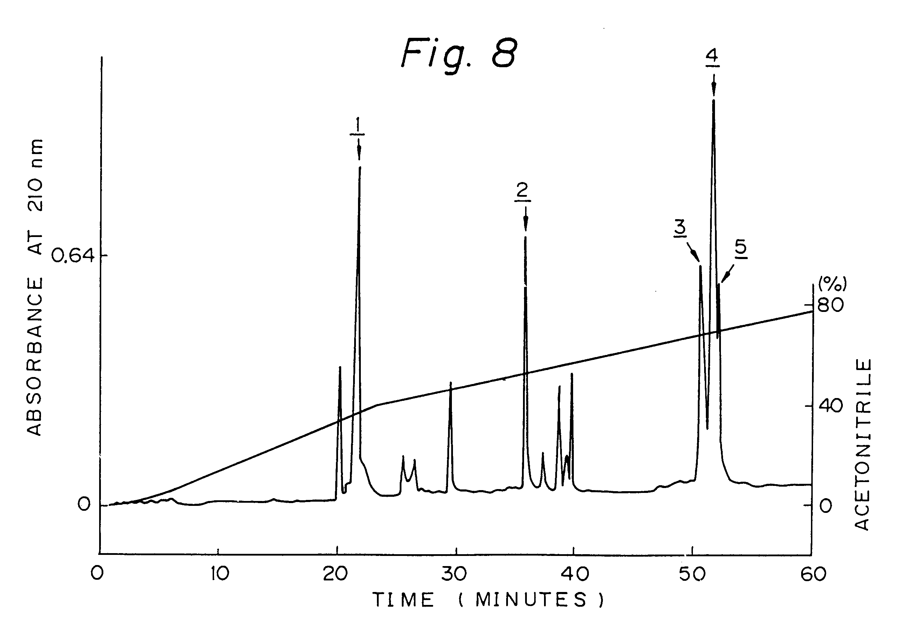

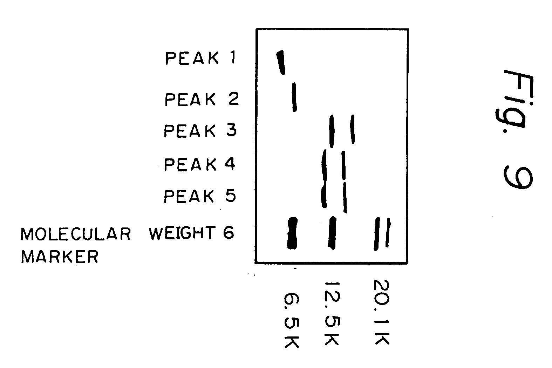

To the solution of sulfonated fused protein obtained in Example 9, was added calf thrombin (Sigma) in an amount of 1/2000 by weight/weight relative to the total protein, and a reaction was carried out at 37°C for 12 hours. The results of the reverse HPLC analysis for an aliquot of the reaction mixture are shown in Fig. 8. Moreover, a fraction of each peak was obtained and analyzed by SDS-PAGE. The results are shown in Fig. 9. The N-terminal amino acid sequence for each fraction was determined using a protein sequencer (Applied Biosystems 470A) and a PTH analyzer (Applied Biosystems 470A). The results are shown in Table 3.

## Table 3

| Peak Number | Cycle Number | | | | | | | | | |
|---|---|---|---|---|---|---|---|---|---|---|
| | 1 | 2 | 3 | 4 | 5 | 6 | 7 | 8 | 9 | 10 |
| 1 | Asn | Pro | Thr | Arg | Arg | Lys | Pro | Gly | Lys | - |
| 2 | Ala | His | Arg | Leu | His | | | | | |
| 3 4 5 | Met Ala | Phe His | Pro Arg | Thr Leu | Ile His | | | | | |
| (Asn$^{55}$-Ala$^{107}$) SLPI | Asn | Pro | Thr | Arg | Arg | Lys | Pro | Gly | Lys | Cys |

It was found from the above results that the desired sulfonated derivative of the (Asn$^{55}$-Ala$^{107}$) SLPI fragment polypeptide corresponds to peak 1. The peak 1 was obtained using a preparative column (Vydac-214 TP1010), and lyophilized to obtain 2 mg of a sulfonated derivative of the (Asn$^{55}$-Ala$^{107}$) SLPI polypeptide fragment.

Example 11. Refolding of sulfonated derivative of (Asn$^{55}$-Ala$^{107}$) SLPI polypeptide fragment into active molecule

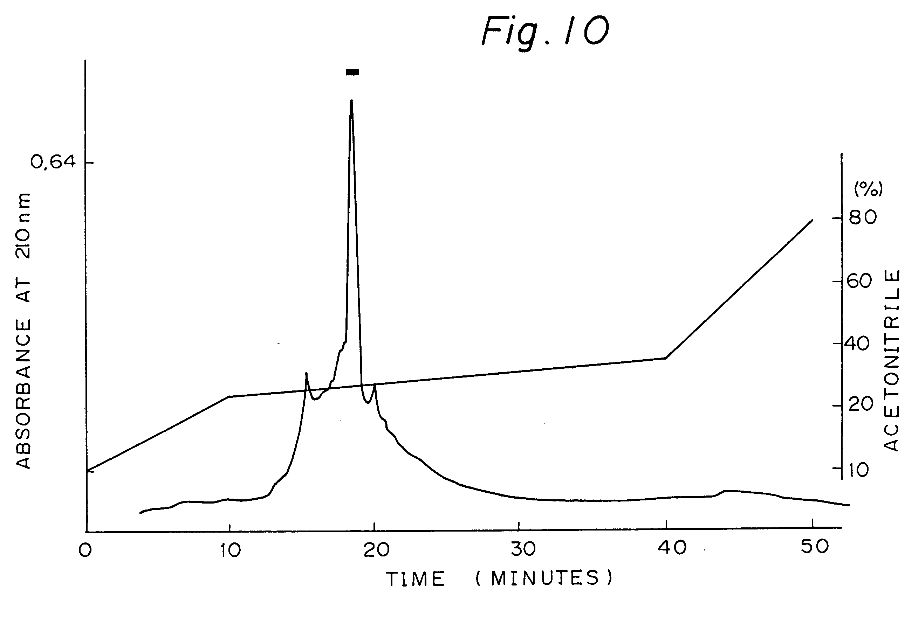

Two mg of a sulfonated derivative of the (Asn$^{55}$-Ala$^{107}$) SLPI polypeptide fragment obtained in Example 10 was dissolved in 1 ml of 50 mM Tris-HCl (pH 8.0), 2-mercaptoethanol was added to the solution to a final concentration of 1%, and a reaction was carried out at 45°C for two hours. To this solution was added 1 ml of 3 M sodium acetate (pH 5.0), and the mixture was put into a dialysis tube and dialyzed against 10 ℓ of a solution containing 50 mM sodium acetate (pH 5.0), 10 $\mu$M oxidized glutathione, and 20 $\mu$M reduced glutathione, and then dialyzed twice against 10 ℓ of a 50 mM Tris-HCl buffer (pH 8.5). The resulting solution was then subjected to reverse HPLC separation to obtain 1 mg of active-type (Asn$^{55}$-Ala$^{107}$) SLPI fragment polypeptide. The HPLC separation pattern is shown in Fig. 10.

Example 12. Assay of serine protease inhibitory activity of active-type (Asn$^{55}$-Ala$^{107}$) SLPI fragment polypeptide

The active-type (Asn$^{55}$-Ala$^{107}$) SLPI fragment polypeptide obtained by refolding by the procedure shown in Example 11 as measured to determine its inhibitory activity to various serine proteases. An assay result thereof is shown as follows.

Buffer: 0.1 M HEPES, 1.0 M NaCl, 0.1% PEG-6000 (pH 7.5)

Enzyme solution: The following enzymes were dissolved in the above-mentioned buffer solutions at a concentration ten-times higher than the concentration shown in Table 4.

(1) Polymorphonuclear leukocyte elastase from human sputum (EPC Co.; Funakoshi Yakuhin)
(2) Calf pancreatic trypsin (Sigma)
(3) Calf pancreatic chymotrypsin (Sigma)
(4) Porcine pancreatic elastase (Sigma)
(5) Human plasma thrombin (Kabi, Daiichi Kagaku Yakuhin)
(6) Human plasma plasmin (Kabi, Daiichi Kagaku Yakuhin)
(7) Human plasma kallikrein (Kabi, Daiichi Kagaku Yakuhin)

Substrate solution: For the above-mentioned enzymes (1) to (7), the following substrates (1) to (7), respectively, were dissolved in dimethyl sulfoxyde to a concentration of 10 mM to prepare stock solutions, which were then dissolved in the above-mentioned buffer solution to concentrations ten-times higher than final concentrations, to prepare substrate solutions.

| Substrate | Final concentration in reaction mixture |
|---|---|
| (1) MeO-Suc-Ala-Ala-Pro-Val-pNA[1] | 0.3 mM |
| (2) Bz-Arg-pNA[1] | 1.0 mM |
| (3) Suc-Ala-Ala-Pro-Phe-pNA[1] | 0.1 mM |
| (4) Suc-Ala-Ala-Ala-pNA[1] | 0.1 mM |
| (5) H-D-Phe-Pip-Arg-pNA[2] | 0.1 mM |
| (6) H-D-Val-Leu-Lys-pNA[2] | 0.1 mM |
| (7) H-D-Pro-Phe-Arg-pNA[2] | 0.1 mM |

1) Backem
2) Kabi; Daiichi Kagaku Yakuhin

To each well of a 96-well ELISA microplate were added 140 $\mu$l of the above-mentioned buffer, 20 $\mu$l of a test solution, and 20 $\mu$l of the enzyme solution, and the mixture was stirred at 37°C for 30 minutes. Next, 20 $\mu$l of the substrate solution was added to the well, the mixture was stirred at 37°C for one hour to develop same, and the absorbance at 405 nm was measured. As inhibitory proteins, an active-type (Asn$^{55}$-Ala$^{107}$) SLPI fragment polypeptide, as well as $\alpha_1$-AT (Sigma), and aprotinin (Boehringer) as a positive control, were used. The inhibitory activities were measured for various concentrations of these proteins to calculate a concentration of inhibitory protein which exhibits a 50% inhibition. The results are shown in Table 4.

Table 4

| Enzyme (concentration) | (Asn$^{55}$-Ala$^{107}$) SLPI | $\alpha_1$-PI | Aprotinin |
|---|---|---|---|
| Polymorphonuclear leucocyte elastase from human sputum ($10^{-9}$ M) | $1 \times 10^{-9}$ | M $3 \times 10^{-9}$ | M $3 \times 10^{-7}$ M |
| Calf pancreatic trypsin ($10^{-7}$ M) | $1.3 \times 10^{-7}$ | M $2 \times 10^{-7}$ | M $1 \times 10^{-7}$ M |
| Calf pancreatic chymotrypsin ($10^{-8}$ M) | $1 \times 10^{-8}$ | M $2 \times 10^{-6}$ | M $5 \times 10^{-8}$ M |
| Porcine pancreatic elastase ($6 \times 10^{-9}$ M) | $2 \times 10^{-7}$ | M $3 \times 10^{-7}$ | M >$2 \times 10^{-6}$ M |
| Human plasma thrombin ($10^{-11}$ M) | >$2 \times 10^{-6}$ | M >$2 \times 10^{-6}$ | M >$2 \times 10^{-6}$ M |
| Human plasma plasmin ($10^{-8}$ M) | >$2 \times 10^{-6}$ | M >$2 \times 10^{-6}$ | M $5 \times 10^{-9}$ M |
| Human plasma kallikrein ($10^{-8}$ M) | >$2 \times 10^{-6}$ | M >$2 \times 10^{-6}$ | M $7 \times 10^{-8}$ M |

Moreover, an inhibitory constant Ki of the present (Asn$^{55}$-Ala$^{107}$) SLPI was calculated on the basis of the above data by a method of Dixon, M. and Webb, E.C. (1979), Enzyme, Longman or a method of Henderson, P.J.F. (1972) Biochem. J., 127, 321-333. The results are shown in Table 5.

Table 5

| Enzyme | Dixon method | Henderson method |
|---|---|---|
| Polymorphonuclear leucocyte elastase from human sputum | $3 \times 10^{-11}$ M | $2 \times 10^{-10}$ M |
| Bovine pancreatic trypsin | $6 \times 10^{-8}$ M | $2 \times 10^{-8}$ M |

As seen from Table 5, a ratio of inhibitory constant to polymorphonuclear leucocyte elastase from human sputum [Ki(E)] and inhibitory constant to calf pancreatic trypsin [Ki(T)], i.e., [Ki(T)/Ki(E)] is 1/100 to 1/1000, revealing that a specificity to elastase is increased in comparison to a native SLPI. Note, in a native SLPI, it is known that Ki(E) is roughly equivalent to Ki(T), R.C. Thompson et al., Proc. Natl. Acad. Sci. USA, 83, 6692 (1980).

Example 13. Effect of serum albumin on inhibitory activity

The inhibitory activity of a active-form (Asn$^{55}$-Ala$^{107}$) SLPI fragment polypeptide on polymorphonuclear leucocyte elastase from human sputum was measured by the same procedure as in Example 12 in the presence of 0.8% or 8% calf serum albumin. The calf serum albumin had no effect on its activity and equivalent inhibitory activities were exhibited.

Example 14. Thermal stability

After a solution of an active-type (Asn$^{55}$-Ala$^{107}$) SLPI fragment polypeptide in 50 mM Tris-HCl (pH 7.8) was treated at 50°C for 2 hours, the inhibitory activity on polymorphonuclear leukocyte elastase from human sputum was measured. The activity was maintained at the same level.

INDUSTRIAL APPLICABILITY

Since the present elastase inhibitory polypeptide exhibits a high elastase inhibitory activity, but a low inhibitory activity to other serine proteases, in particular trypsin-like serine protease, it is promising as a useful therapeutic agent for the suppression of the progress of emphyseme.

Further, the present fused protein expression system using a human growth hormone or a portion thereof is highly efficient and can be universally used to produce a relatively low molecular peptide by gene recombination.

**Claims**

1. An elastase inhibitory polypeptide or homopolymer thereof having an amino acid sequence represented by the formula (I):

$$X-(-Asn-Pro-Thr-Arg-Arg-Lys-Pro-Gly-Lys-Cys-Pro-Val-$$

$$Thr-Tyr-Gly-Gln-Cys-Leu-Met-Leu-Asn-Pro-Pro-Asn-Phe-$$

$$Cys-Glu-Met-Asp-Gly-Gln-Cys-Lys-Arg-Asp-Leu-Lys-$$

$$Cys-Cys-Met-Gly-Met-Cys-Gly-Lys-Ser-Cys-Val-Ser-$$

$$Pro-Val-Lys-Ala-)_n$$

$$(I)$$

or a part of the formula (I), wherein one or more than one amino acid is deleted from the N-terminal of the amino acid sequence in parentheses, wherein X represents Gly or is absent and n represents an integer of 1 to 10, characterised in that the trypsin-like serine protease inhibitory activity of the polypeptide does not exceed 1/100 of the elastase inhibitory activity.

2. An elastase inhibitory polypeptide or homopolymer thereof according to claim 1 wherein the trypsin-like serine protease inhibitory activity of the polypeptide does not exceed 1/1000 of the elastase inhibitory activity.

3. An elastase inhibitory polypeptide or homopolymer thereof according to claim 1 or 2, wherein 1 to 8 cysteine residues are sulphonated.

4. A fused protein represented by the formula (II):

Y - B - Z    (II)

wherein Y presents a carrier protein comprising a human growth hormone or a fragment thereof;
Z represents an elastase inhibitory polypeptide or homopolymer thereof according to claim 1 or 2; and
B represents a linking peptide or polypeptide having an amino acid sequence which can be chemically or biologically cleaved under conditions wherein the desired protein is not denatured; wherein B is joined to amino terminus Asn in Z.

5. A fused protein according to claim 4, wherein in the formula (II), 1 to 8 cysteine residues are sulphonated.

6. A fused protein according to claim 4, wherein the carrier protein is a polypeptide fragment from the first amino acid residue Phe to the 139th amino acid residue Phe of a native human growth hormone.

7. A fused protein according to claim 4, wherein the carrier protein is a polypeptide fragment from the first amino acid residue Phe to the 122th amino acid residue Gln of a native human growth hormone.

8. A fused protein according to claim 4, wherein B is a linking peptide or polypeptide having an amino acid sequence selected from the group consisting of

$$-(-Asn-Gly-)_n \ , \ -(-Val-Pro-Arg-)_n \ ,$$

and

$$-(-Leu-Val-Pro-Arg-)_n$$

wherein n represents an integer of 1 to 10, and Asn at an amino terminus in Z is joined to Gly, Arg or Arg in B respectively.

9. A process for production of an elastase inhibitory polypeptide or homopolymers thereof represented by the formula (I) in claim 1, wherein X represents Gly, comprising the step of treating a fused protein represented by the formula (IV:

$$Y \underleft( Asn\text{-}Gly \underright)_n Z \qquad\qquad (IV)$$

wherein Y and Z have the meanings defined under the formula (II), n represents an integer of 1 to 10, and Gly in the formula (IV) is linked to an N-terminal Asn in Z, with hydroxylamine or an analog thereof.

10. A process for production of a polypeptide or homopolymers thereof represented by the formula (I) in claim 1, wherein X is absent, comprising the step of treating a fused protein represented by the formula (V):

Y - B' - Z    (V)

wherein Y and Z have meanings as defined under the formula (II), B' represents

$$\underleft( Val\text{-}Pro\text{-}Arg \underright)_n \text{ or } \underleft( Leu\text{-}Val\text{-}Pro\text{-}Arg \underright)_n$$

wherein n represents an integer of 1 to 10, and Asn in Y is linked to Arg in B' by thrombin or an analog thereof.

11. A fused protein gene comprising a gene coding for a human hormone or a fragment thereof as a carrier protein linked to a gene coding for a desired protein or a segment thereof via a gene coding for a linking peptide or homopolymer thereof having an amino acid sequence which can be chemically or biologically cleaved under a condition wherein the desired protein is not denatured.

12. A gene according to claim 11, wherein the desired protein or protein thereof is an elastase inhibitory polypeptide having an amino acid sequence represented by the formula (III), or a protein thereof exhibiting an elastase inhibitory activity or an amino acid sequence biologically equivalent thereto.

13. A fused protein gene according to claim 11, wherein the amino acid sequence which can be chemically or biologically cleaved under the condition wherein the desired protein is not denatured is

$$\underleft( Asn\text{-}Gly \underright)_n, \underleft( Val\text{-}Pro\text{-}Arg \underright)_n, \text{ or } \underleft( Leu\text{-}Val\text{-}Pro\text{-}Arg \underright)_n$$

wherein n represents a integer of 1 to 10.

14. A fused protein gene according to claim 11, wherein the carrier protein is a polypeptide from the first amino acid residue Phe to the 139th amino acid residue Phe of a native human growth hormone.

15. A plasmid for expression of a fused protein gene comprising the fusion protein gene according to any one of claims 11 to 15.

16. A transformed microbial cell carrying the fused protein gene according to any one of claims 11 to 14.

**Patentansprüche**

1. Elastase inhibierendes Polypeptid oder Homopolymer hiervon mit einer Aminosäuresequenz gemäß Formel (I):

```
X-(-Asn-Pro-Thr-Arg-Arg-Lys-Pro-Gly-Lys-Cys-Pro-Val-

   Thr-Tyr-Gly-Gln-Cys-Leu-Met-Leu-Asn-Pro-Pro-Asn-Phe-

   Cys-Glu-Met-Asp-Gly-Gln-Cys-Lys-Arg-Asp-Leu-Lys-        (I)

   Cys-Cys-Met-Gly-Met-Cys-Gly-Lys-Ser-Cys-Val-Ser-

   Pro-Val-Lys-Ala-)$_n$-
```

oder einem Teil der Formel (I), worin eine oder mehr als eine Aminosäure vom N-ständigen Ende der in Klammern stehenden Aminosäuresequenz entfernt ist, worin X Gly repräsentiert oder fehlt und n eine ganze Zahl von 1 bis 10 repräsentiert, dadurch gekennzeichnet, daß die Trypsin-ähnliche Serin-Protease inhibierende Aktivität des Polypeptids 1/100 der Elastase inhibierenden Aktivität nicht übersteigt.

2. Elastase inhibierendes Polypeptid oder Homopolymer hiervon entsprechend Anspruch 1, worin die Trypsin-ähnliche Serin-Protease inhibierende Aktivität des Polypeptids 1/1000 der Elastase inhibierenden Aktivität nicht übersteigt.

3. Elastase inhibierendes Polypeptid oder Homopolymer hiervon gemäß Anspruch 1 oder 2, worin 1 bis 8 Cystein-Reste sulfoniert sind.

4. Verschmolzenes Protein entsprechend der Formel (II):

   Y - B - Z     (II)

   worin Y ein Trägerprotein, welches ein menschliches Wachstumshormon oder ein Fragment hiervon enthält, repräsentiert;
   worin Z ein Elastase inhibierendes Polypeptid oder ein Homopolymer hiervon gemäß Anspruch 1 oder 2 repräsentiert; und
   B ein Kopplungspeptid oder -polypeptid mit einer Aminosäuresequenz repräsentiert, welche chemisch oder biologisch unter Bedingungen spaltbar ist, unter denen das Protein nicht denaturiert wird; worin B mit Asn am Aminoende in Z verknüpft ist.

5. Verschmolzenes Protein nach Anspruch 4, worin in der Formel (II) 1 bis 8 Cystein-Reste sulfoniert sind.

6. Verschmolzenes Protein nach Anspruch 4, worin das Trägerprotein ein Polypeptidfragment vom ersten Aminosäurerest Phe bis zum 139. Aminosäurerest Phe eines natürlich vorkommenden menschlichen Wachstumshormons ist.

7. Verschmolzenes Protein nach Anspruch 4, worin das Trägerprotein ein Polypeptidfragment vom ersten Aminosäurerest Phe bis zum 122. Aminosäurerest Gln eines natürlich vorkommenden menschlichen Wachstumshormons ist.

8. Verschmolzenes Protein nach Anspruch 4, worin B ein Kopplungspeptid oder -polypeptid mit einer aus der aus -(-Asn-Gly-)$_n$- , -(-Val-Pro-Arg-)$_n$- und -(-Leu-Val-Pro-Arg-)$_n$- bestehenden Gruppe ausgewählten Aminosäuresequenz ist, worin n eine ganze Zahl von 1 bis 10 ist und Asn an einem Aminoende in Z mit Gly, Arg bzw. Arg in B verknüpft ist.

9. Verfahren zur Herstellung eines Elastase inhibierenden Polypeptids oder Homopolymeren hiervon, entsprechend der Formel (I) in Anspruch 1, worin X Gly repräsentiert, umfassend den Schritt der Behandlung eines verschmolzenen Proteins gemäß der Formel (IV):

   Y-(-Asn-Gly-)$_n$- Z     (IV)

   worin Y und Z die Bedeutung, wie im Zusammenhang mit der Formel (II) definiert, haben, n eine ganze Zahl von 1 bis 10 ist und worin Gly in der Formel (IV) mit einem N-endständigen Asn in Z verknüpft ist,

20

mit Hydroxylamin oder einem Analogen hiervon.

10. Verfahren zur Herstellung eines Polypeptids oder eines Homopolymeren hiervon, entsprechend Formel (I) aus Anspruch 1, worin X fehlt, umfassend den Schritt der Behandlung eines verschmolzenen Proteins gemäß der Formel (V):

Y - B' - Z      (V)

worin Y und Z die Bedeutungen aufweisen, wie sie für Formel (II) definiert wurden, worin B' -(-Val-Pro-Arg-)$_n$-oder -(-Leu-Val-Pro-Arg)$_n$- repräsentiert, wobei n eine ganze Zahl von 1 bis 10 ist, und worin Asn in Z mit Arg in B' mittels Thrombin oder einem Analogen hiervon verknüpft ist.

11. Gen für ein verschmolzenes Protein, umfassend ein für ein als Trägerprotein fungierendes menschliches Hormon oder ein Fragment hiervon kodierendes Gen, welches mit einem für ein gewünschtes Protein oder ein Teil hiervon kodierenden Gen über ein ein Kopplungspeptid oder Homopolymer hiervon kodierenden Gen verknüpft ist, wobei das Kopplungspeptid oder das Homopolymer hiervon eine Aminosäurensequenz aufweist, welche chemisch oder biologisch unter einer Bedingung spaltbar ist, unter der das gewünschte Protein nicht denaturiert wird.

12. Gen nach Anspruch 11, worin das Protein oder Protein hiervon ein Elastase inhibierendes Polypeptid mit einer Aminosäuresequenz gemäß Formel (III) oder einem Teil hiervon ist, welches eine Elastase inhibierende Aktivität zeigt, oder eine biologisch hierzu äquivalente Aminosäuresequenz.

13. Gen für ein verschmolzenes Protein nach Anspruch 11, worin die Aminosäuresequenz, welche chemisch oder biologisch unter der Bedingung spaltbar ist, unter der das gewünschte Protein nicht denaturiert wird, -(-Asn-Gly-)$_n$- , -(-Val-Pro-Arg)$_n$- oder -(-Leu-Val-Pro-Arg-)$_n$- ist, wobei n eine ganze Zahl von 1 bis 10 ist.

14. Gen für ein verschmolzenes Protein nach Anspruch 11, worin das Trägerprotein ein Polypeptid von dem ersten Aminosäurerest Phe bis zum 139. Aminosäurerest Phe eines natürlich vorkommenden menschlichen Wachstumshormons ist.

15. Plasmid zur Expression eines Gens für ein verschmolzenes Protein, umfassend das Gen für ein verschmolzenes Protein gemäß einem der Ansprüche 11 bis 14.

16. Transformierte Zelle eines Mikroorganismus, welche das Gen für ein verschmolzenes Protein gemäß einem der Ansprüche 11 bis 14 trägt.

**Revendications**

1. Polypeptide inhibiteur d'élastase ou homopolymère de celui-ci ayant une séquence d'acides aminés représentée par la formule (I):

$$X—(—Asn–Pro–Thr–Arg–Arg–Lys–Pro–Gly–Lys–Cys–Pro–Val–$$
$$Thr–Tyr–Gly–Gln–Cys–Leu–Met–Leu–Asn–Pro–Pro–Asn–Phe–$$
$$Cys–Glu–Met–Asp–Gly–Gln–Cys–Lys–Arg–Asp–Leu–Lys–$$
$$Cys–Cys–Met–Gly–Met–Cys–Gly–Lys–Ser–Cys–Val–Ser–$$
$$Pro–Val–Lys–Ala—)_n—$$

$$(I)$$

ou par une partie de la formule (I), où un ou plusieurs acides aminés sont délétés de l'extrémité N-terminale de la séquence d'acides aminés entre parenthèses, X représente Gly ou est absent et n représente un nombre entier de 1 à 10, caractérisé en ce que l'activité inhibitrice de protéase à sérine de type trypsine du polypeptide ne dépasse pas 1/100 de l'activité inhibitrice d'élastase.

2. Polypeptide inhibiteur d'élastase ou homopolymère de celui-ci selon la revendication 1, dans lequel l'activité inhibitrice de protéase à sérine de type trypsine du polypeptide ne dépasse pas 1/1000 de l'activité inhibitrice d'élastase.

3. Polypeptide inhibiteur d'élastase ou homopolymère de celui-ci selon la revendication 1 ou 2, dans lequel 1 à 8 résidus cystéine sont sulfonés.

4. Protéine de tusion représentée par la formule (II):

Y-B-Z    (II)

dans laquelle Y représente une protéine porteuse comprenant une hormone de croissance humaine ou un fragment de celle-ci,
Z représente un polypeptide inhibiteur d'élastase ou un homopolymère de celui-ci selon la revendication 1 ou 2, et
B représente un peptide ou polypeptide de liaison ayant une séquence d'acides aminés qui peut être clivée chimiquement ou biologiquement dans des conditions dans lesquelles la protéine voulue n'est pas dénaturée, où B est lié à l'Asn aminoterminale de Z.

5. Protéine de fusion selon la revendication 4, dans laquelle, dans la formule (II), 1 à 8 résidus cystéine sont sulfonés.

6. Protéine de fusion selon la revendication 4, dans laquelle la protéine porteuse est un fragment polypeptidique du premier résidu d'acide aminé Phe au $139^e$ résidu d'acide aminé Phe d'une hormone de croissance humaine native.

7. Protéine de fusion selon la revendication 4, dans laquelle la protéine porteuse est un fragment polypeptidique du premier résidu d'acide aminé Phe au $122^e$ résidu d'acide aminé Gln d'une hormone de croissance humaine native.

8. Protéine de fusion selon la revendication 4, dans laquelle B est un peptide ou polypeptide de liaison ayant une séquence d'acides aminés choisie dans le groupe consistant en :

$$-(-Asn-Gly-)_n-, \quad -(-Val-Pro-Arg-)_n-$$

et

$$-(-Leu-Val-Pro-Arg-)_n-$$

où n représente un nombre entier de 1 à 10 et Asn à l'extrémité amino-terminale de Z est liée à Gly, Arg ou Arg de B respectivement.

9. Procédé de production d'un polypeptide inhibiteur d'élastase ou d'homopolymères de celui-ci représentés par la formule (I) de la revendication 1, dans laquelle X représente Gly, comprenant l'étape de traitement d'une protéine de fusion représentée par la formule (IV):

$$Y-(-Asn-Gly-)_n-Z \quad\quad (IV)$$

dans laquelle Y et Z ont les significations définies à propos de la formule (II), n représente un nombre entier de 1 à 10 et Gly dans la formule (IV) est liée à une Asn N-terminale de Z, avec l'hydroxylamine ou un analogue de celle-ci.

10. Procédé de production d'un polypeptide ou d'homopolymères de celui-ci représentés par la formule (I) de la revendication 1, dans laquelle X est absent, comprenant l'étape de traitement d'une protéine de fusion représentée par la formule (V):

Y-B'-Z    (V)

dans laquelle Y et Z ont les significations définies à propos de la formule (II), B' représente

$$-(\!\!-Val\!-\!Pro\!-\!Arg\!-\!)_n\!- \text{ ou } -(\!\!-Leu\!-\!Val\!-\!Pro\!-\!Arg\!-\!)_n\!-$$

où n représente un nombre entier de 1 à 10 et Asn de Z est liée à Arg de B', par la thrombine ou un analogue de celle-ci.

11. Gène de protéine de fusion comprenant un gène codant une hormone humaine ou un fragment de celle-ci en tant que protéine porteuse lié à un gène codant une protéine voulue ou un fragment de celle-ci par l'intermédiaire d'un gène codant un peptide de liaison ou un homopolymère de celui-ci ayant une séquence d'acides aminés qui peut être clivée chimiquement ou biologiquement dans des conditions dans lesquelles la protéine voulue n'est pas dénaturée.

12. Gène selon la revendication 11, dans lequel la protéine voulue ou un fragment de celle-ci est un polypeptide inhibiteur d'élastase ayant une séquence d'acides aminés représentée par la formule (III) ou une partie de celle-ci présentant une activité inhibitrice d'élastase, ou une séquence d'acides aminés biologiquement équivalente à celle-ci.

13. Gène de protéine de fusion selon la revendication 11, dos lequel la séquence d'acides aminés qui peut être clivée chimiquement ou biologiquement dans des conditions dans lesquelles la protéine voulue n'est pas dénaturée est

$$-(\!\!-Asn\!-\!Gly\!-\!)_n\!-, \qquad -(\!\!-Val\!-\!Pro\!-\!Arg\!-\!)_n\!-$$

ou

$$-(\!\!-Leu\!-\!Val\!-\!Pro\!-\!Arg\!-\!)_n\!-$$

où n représente un nombre entier de 1 à 10.

14. Gène de protéine de fusion selon la revendication 11, dans lequel la protéine porteuse est un polypeptide du premier résidu d'acide aminé Phe au $139^e$ résidu d'acide aminé Phe d'une hormone de croissance humaine native.

15. Plasmide pour l'expression d'un gène de protéine de fusion comprenant le gène de protéine de fusion selon l'une quelconque des revendications 12 à 15.

16. Cellule microbienne transformée portant le gène de protéine de fusion selon l'une quelconque des revendications 11 à 14.

# Fig. 1

SGKSFKAGVCPPKKSAQCLRYKKPECQSDWQCPGKKRCCPDTCGIKCLDPVDTP

NPTRRKPGKCPVTYGQCLMLNPPNFCEMDGQCKRDLKCCMGMCGKSCVSPVKA

# Fig. 2

<u>BamHI</u>  <u>SalI</u>                        <u>MluI</u>
GATCCGGTCGACACCCCGAACCCGACGCGTCGT
       GCCAGCTGTGGGGCTTGGGCTGCGCAGCA
                          AsnProThrArgArg

                 <u>NdeI</u>
AAACCGGGTAAATGTCCGGTTACATATGGTCAGTGTCTGATGCTGAACCCGCCGAACTTC
TTTGGCCCATTTACAGGCCAATGTATACCAGTCACAGACTACGACTTGGGCGGCTTGAAG
LysProGlyLysCysProValThrTyrGlyGlnCysLeuMetLeuAsnProProAsnPhe

                 <u>BglII</u>
TGTGAAATGGACGGTCAGTGTAAACGAGATCTGAAATGTTGTATGGGTATGTGTGGTAAA
ACACTTTACCTGCCAGTCACATTTGCTCTAGACTTTACAACATACCCATACACACCATTT
CysGluMetAspGlyGlnCysLysArgAspLeuLysCysCysMetGlyMetCysGlyLys

                 <u>XhoI</u>  <u>PstI</u>
TCTTGTGTTTCTCCGGTTAAAGCATAATAGCTCGAGCTGCA
AGAACACAAAGAGGCCAATTTCGTATTATCGAGCTCG
SerCysValSerProValLysAlastopstop

*Fig. 3*

```
                              5'
                   ① 5'  GATCCGGTCGACACCCCGAACCCGACGCGTCGT
                        ②  GCCAGCTGTGGGGCTTGGGCTGCGCAGCA
                     3'
```

```
                                                ③
AAACCGGGTAAATGTCCGGTTACATATGGTCAGTGTCTGATGCTGAACCCGCCGAACTTC
TTTGGCCCATTTACAGGCCAATGTATACCAGTCACAGACTACGACTTGGGCGGCTTGAAG
                                                ④
```

```
                                              ⑤
TGTGAAATGGACGGTCAGTGTAAACGAGATCTGAAATGTTGTATGGGTATGTGTGGTAAA
ACACTTTACCTGCCAGTCACATTTGCTCTAGACTTTACAACATACCCATACACACCATTT
                                                    ⑥
```

```
                                                    3'
TCTTGTGTTTCTCCGGTTAAAGCATAATAGCTCGAGCTGCA
AGAACACAAAGAGGCCAATTTCGTATTATCGAGCTCG
                                            5'
```

*Fig. 4*

THROMBIN CLEVAGE SITE

```
Gln Ile Phe Leu Val Pro Arg Asn Pro Thr
5'
⑦  G-ATC-TTC-CTG-GTT-CCG-CGT-AAC-CCG-A
      ⑧  AAG-GAC-CAA-GGC-GCA-TTG-GGC-TGC-GC
        3'
    _____                              _____
    BglII                                MluI
```

HYDROXYLAMINE CLEAVAGE SITE

```
Gln Ile Phe Asn Gly Asn Pro Thr Arg
5'
⑨  G-ATC-TTC-AAC-GGT-AAC-CCG-A
      ⑩ -AAG-TTG-CCA-TTG-GGC-TGC-GC
        3'
    _____                        _____
    BglII                          MluI
```

25

# Fig. 5

## Fig. 6

1 MOLECULAR WEIGHT MARKER

2 POLYPEPTIDES FROM E.COLI HB101

3 POLYPEPTIDES FROM E.COLI HB101
     (pGH-TE)

4 POLYPEPTIDES FROM E.COLI HB101
     (pGH-HE)

## Fig. 7

1 MOLECULAR WEIGHT MARKER

2 POLYPEPTIDES FROM E.COLI HB101 (pGH-TE)

3 POLYPEPTIDES FROM E.COLI HB101 (pGH-TE)
                           (THROMBIN 2 HOURS)

4 POLYPEPTIDES FROM E.COLI HB101 (pGH-TE)
                           (THROMBIN 15 HOURS)

5 POLYPEPTIDES FROM E.COLI HB101 (pGH-HE)

6 POLYPEPTIDES FROM E.COLI HB101 (pGH-HE)
                           (HYDROXYLAMINE 1 HOURS)

7 POLYPEPTIDES FROM E.COLI HB101 (pGH-HE)
                           (HYDROXYLAMINE 4 HOURS)

Fig. 8

EP 0 346 500 B1

Fig. 9

# Fig. 10

Fig. 11